**Europäisches Patentamt**

**European Patent Office**

**Office européen des brevets**

(19)

(11) **EP 1 287 746 A1**

(12) **EUROPEAN PATENT APPLICATION**

(43) Date of publication:
**05.03.2003 Bulletin 2003/10**

(51) Int Cl.⁷: **A23G 3/30**

(21) Application number: **01119703.5**

(22) Date of filing: **24.08.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **THE PROCTER & GAMBLE COMPANY
Cincinnati, Ohio 45202 (US)**

(72) Inventors:
• **Gonzales, Denis
65100 Pescara (IT)**

• **Gagliardi, Ivano
65123 Pescara (IT)**
• **Carlucci, Giovanni
66100 Chieti (IT)**
• **Dipilla, Mario
65100 Pescara (IT)**

(74) Representative: **Kremer, Véronique et al
Procter & Gamble Service GmbH
65823 Schwalbach am Taunus (DE)**

(54) **Chewable compositions with odour absorbing agents**

(57) The present invention relates to a chewable composition comprising a chewable base and an odour absorbing agent or combination thereof. The chewable compositions allow effective reduction of bad breath.

**EP 1 287 746 A1**

**Description**

Field of the Invention

The present invention relates to chewable compositions and more particularly to chewable confectionery compositions for controlling odours, especially bad breath, said compositions comprising an odour absorbing agent or combination thereof.

Background of the Invention

In the oral care field, bad breath is rated by consumers as being one of the most frequent oral problems beside tartar build-up. In most of the case this problem originates from the mouth cavity due to either poor oral hygiene, or from food odor or microbial metabolism of food and/or oral infections.

This problem is traditionally solved per using conventional brushing or rinsing with dentifrice or by masking of the odor per using flavored means like candy.

There is thus a continuous need to overcome bad breath per oral treatment in an easy way compatible with active life. This need has not been satisfied today, especially not the need to get bad odor control upon prolonged periods of time.

It is thus an object of the present invention to provide a means suitable to be used easily at any moment of consumer life to control bad breath. More particularly it is a further object of the present invention to provide a means suitable for easy daily use in a situation of modern active life that does not only deliver immediate odor control properties but is also able to control odor upon prolonged period of use, this while not exhibiting any detrimental particular side effect.

These objects have now surprisingly been met by providing a chewable composition comprising a chewable base and an odor-absorbing agent or combination thereof.

Indeed the presence of an odor absorbing agent, preferably zeolite or cyclodextrin alone, more preferably mixtures of any odor absorbing agent described herein after, and most preferably zeolite or cyclodextrin in combination together or with another odor absorbing agent, in a chewable composition, allows the control of bad breath by absorbing the volatile compound responsible for bad odor. Indeed, these odor absorbing agents have been found to be particularly effective towards various undesirable odorous compounds like volatile sulfur compounds (VSC) (like hydrogen sulfide, methyl mercaptan, dimethyl sulfide and dimethyl disulfide, of which hydrogen sulfide is a major component), amine derivatives (like putrescine, cadaverine, etc,), alliceous compounds (like onions garlic (allyl disulfide, allyl mercaptam, etc.), coffee (e.g., 2-methylbutyraldehyde, etc.), fatty compounds (like acetanisole, acetoin, etc.), cheese (e.g., anisole, benzylbenzoate, etc.), sour (e.g., 2-ethyl butyric acid, isovaleraldehyde, etc.), oily compounds (e.g., allyl 2-ethylbutyrate etc.), meaty (e.g., indole, skatole, allyl-alpha-ionone, benzyl mercaptan, etc.), smoke or cigarette (e.g., 4-Ethylguaiacol, furfuryl mercaptan, etc.), spicy (e.g., cinnamic acid, etc.), wine (e.g., allyl nonanoate, amyl octanoate) and the like.

Advantageously, in contrast to commercially available chewable compositions, which traditionally aim only on masking malodor per flavoring agent release, the chewable compositions of the present invention are able to adsorb the volatile compounds responsible for the bad breath.

Indeed, it has been found that headspace concentration of malodorous compounds in the mouth area (i.e., the concentration of malodorous volatiles (or gas) in the mouth' space) is significantly reduced with the chewable compositions of the present invention in use, as compared to the same chewable compositions but in absence of any odor absorbing agent as described herein after.

The use of the odor controlling chewable compositions of the present invention complies with customers' habits since chewable compositions have been culturally well accepted and present the undeniable benefit of being users friendly and portable as opposed to other oral "fresh breath" treatments such as mouth rinsing or brushing solutions.

Advantageously the chewable compositions of the present invention offer effective odor control properties mainly for the following reasons. Without to be bound by any theory, it is speculated that in comparison to other oral treatments like mouthwashes, toothpaste, etc., chewable compositions have a longer time residency in the mouth, thus participating to the long lasting odor control effect. But more importantly the long lasting effect is obtained per the selection of the malodour odor control material used herein, namely the odor absorbing agent as described herein and combination thereof. More particularly, it is believed that the advantage of the chewable compositions, namely chewing gums, is that the mastication/chewing allows a continuous transport of the malodorous compounds (either from the saliva in which it may be dissolved, or from the headspace) toward the odor-absorbing agent contained inside the chewing gum matrix. The mechanism occurs either via chemisorption (or chemical reaction) or physisorption or both. The kinetic of the whole process is favored by the always modified surface of the chewing gum matrix exposing in a more efficient way the odor absorbing agent and the malodorous compounds.

Advantageously the controlled release of odor absorbing agents in the mouth triggered per mastication/chewing results in odor control not only in the mouth area but also in the digestive apparel, thereby extending further the adsorption properties of the odor absorbing agent used in the chewable compositions of the present invention and potentially also the release of actives (e.g., flavors, sweeteners, nutritional or pharmaceutical actives of the like) in the case the chewable compositions further comprise cyclodextrin/active inclusion complexes.

In a particular embodiment of the present invention, the odor-absorbing agent or combination thereof (e.g., zeolite or mixture of zeolite and silica) can replace totally or in part the fillers usually used in the chewing gum matrix (used to adjust the chewing gum consistency), thereby not altering the intrinsic properties of the chewing gum, nor adding to material cost.

In a preferred embodiment the chewable compositions comprise (a) a first type of odor absorbing agent typically selected from the group consisting of zeolite-, silica-based material, uncomplexed cyclodextrin or derivative thereof, carbon-based odor absorbing material (preferably a zeolite-based material) together with (b) a second type of odor absorbing agent typically selected from the group consisting of zeolite-, silica-based material, uncomplexed cyclodextrin or derivative thereof, carbon-based odor absorbing material (preferably a silica-based material), typically in weight ratio of (a) to (b) of 1:5 to 5:1, as the odor absorbing agents, provided (a) and (b) are chosen from different chemical classes of odor-absorbing agents. This combination is highly preferred herein as it has been found that it results in synergistic odor control of bad breath. Indeed, the odor reduction observed with a chewable composition according to the present invention comprising (a) such a first odor absorbing agent (preferably a zeolite-based material) together with (b) such a second odor absorbing agent (preferably a silica-based material), in weight ratio of (a) to (b) of 1:5 to 5:1, is significant higher than the one observed with the same chewable composition but comprising only one of the two odor absorbing agent classes at same total level of odor absorbing agents.

In a preferred embodiment herein the chewable composition further comprises on top of the odor absorbing agent or mixture thereof, a cyclodextrin/active inclusion complex. Indeed the presence of such additional ingredients allows slow release of actives that might be desired in the chewable compositions, including but not limited to, flavors, sweeteners, nutritional or pharmaceutical actives and the like.

Indeed in a particular embodiment, the chewable compositions comprise, beside the odor absorbing agent, a cyclodextrin/flavoring agent inclusion complex. This particular combination results in long lasting odour control per the combination of two different mechanisms of action for controlling malodour, namely (1) reducing malodour per the use of odour absorbing agent and (2) masking residual persistent malodour per release of flavouring agent. Advantageously the presence of the cyclodextrin/flavoring agent inclusion complex also fulfils the function of malodour-control signal.

### Summary of the Invention

The present invention relates to chewable compositions comprising a chewable base and an odour-absorbing agent.

### Detailed description of the Invention

### The odour absorbing agents

The compositions according to the present invention comprise as an essential element, at least one odour absorbing agent or a mixture thereof.

Suitable odour absorbing agents for use herein include zeolite-based material, silica-based materials, uncomplexed cyclodextrin or derivative thereof, carbon-based odor absorbing material, ion exchange resins and the like. Especially preferred are cyclodextrins, especially beta-cyclodextrin, and zeolite material having "intermediate" silicate/aluminate ratios. Without entering into details, the choice of the odor absorbing agent and combination thereof is related to the malodor that is targeted as well to considerations regarding the chewing gum texture (affinity with the gum base formulation) and aesthetics. Particularly suitable combination of odor absorbing agents for the control of bad breath include any mixture of above mentioned odor absorbing agents, especially cyclodextrin or derivative thereof together with either silica-, zeolite-based material, and/or carbon-based odor absorbing agent, or a mixture of zeolite-based material together with silica- and/or carbon-based material.

Highly preferred combination for the control of bad breath is silica-based material together with zeolite-based material.

The chewable compositions typically comprise an odor absorbing agent or mixture thereof at a level of from 1% to 70% by weight of the chewable composition, preferably from 3% to 30% and more preferably from 5% to 15%. In case of mixtures of odor absorbing agents the respective concentration of each agent varies from 0% to 100% of the concentration of the total odor absorbing agents.

Zeolite-based material

The manufacture of zeolite materials of the type used in the practice of this invention is well known, and reference can be made to the voluminous literature for typical synthetic procedures.

In order to assist the formulator and user of the compositions of this invention (but not by way of limitation), attention is directed to the synthetic procedures described in the following reference texts: ZEOLITE SYNTHESIS, ACS Symposium Series 398, Eds. M. L. Occelli and H. E. Robson (1989) pages 2-7; ZEOLITE MOLECULAR SIEVES, Structure, Chemistry and Use, by D. W. Breck, John Wiley b Sons (1974) pages 245-250, 313-314 and 348--352; MODERN APPLICATIONS OF MOLECULAR SIEVE ZEOLITES, Ph.D. Dissertation of S. M. Kuznicki, U. of Utah (1980), available from University Microfilms International, Ann Arbor, Michigan, pages 2-8.

It is preferred (but not necessary) that the zeolites used herein be substantially hydrophobic, since they generally must function to adsorb odors in the presence of saliva in the chewable compositions herein. Moreover, it is preferred (but not necessary) that the potential swelling of zeolites be controlled so to match the feel comfort and aesthetic criteria that the formulator would wish to reach.

While some naturally occurring zeolites meet the objectives of this invention, the synthetic zeolites of the types available in commerce are generally more preferred.

In general terms, traditional zeolites comprise an aluminate/ silicate framework, with associated cations, M, providing overall electrical neutrality. Empirically, the zeolite framework can be represented as

$$x \, AlO_2 \cdot y \, SiO_2$$

and the electrical neutral zeolite as

$$x/n \, M \cdot x \, AlO_2 \cdot y \, SiO_2 \cdot z \, H_2O$$

wherein: x and y are each integers, M is a cation and n is the charge on the cation. As noted by the empirical formula, zeolites may also comprise waters of hydration (z H2O). Reference tQ the literature will illustrate that M can be a wide variety of cations, e.g., Na+, K+, NH,+, alkylammonium, heavy metals and the like. The practice of the present invention does not require any particular selection of cation; accordingly, sodium ion is convenient and preferred.

Preferred zeolites for use herein are crystalline aluminosilicates of group IA and group IIA elements such as Na, K, Mn, Ca and are chemically represented by the empirical formula :

$$M_{2/n}O \cdot Al_2O_3 \cdot ySiO_2 \cdot wH_2O$$

where y is 2 or greater, n is the cation valence, and w is the water content in the voids of the zeolite.

Structurally, zeolites are complex, crystalline inorganic polymers based on an infinitely extending framework of $AlO_4$ and $SiO_4$ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

$$M_{x/n} \, [(AlO_2)_x \, (SiO_2)_y] \cdot wH_2O$$

where n is the valence of cation M, w is the number of water molecules per unit cell, x and y are the total number of tedrahedra per unit cell, y/x usually having values of 1-5.

The synthetic zeolites being preferred for use herein include, but are not limited to for example zeolite A, zeolite P, zeolite Y, zeolite X, zeolite DAY, zeolite ZSM-5, or mixtures thereof.

According to the present invention the zeolite is preferably hydrophobic. This is typically achieved by increasing the molar ratio of the $SiO_2$ to $AlO_2$ content such that the ratio of x to y is at least 1, preferably from 1 to 500, most preferably from 1 to 6.

In an embodiment herein the ratio of integers x and y in this first class of zeolites is such that the zeolites are typically characterized as "intermediate" silicate/al uminate zeolites, whereas those described for example in U.S. 4,795,482 and 4,826,497 are 'high' silicate/aluminate zeolites.

While not intending to be limited by theory, it appears that the silicate/aluminate ratios of the "intermediate" zeolites used in the practice of this invention result in several advantages over the "high" zeolites. First, the intermediate zeolites have a higher capacity for amine-type odors than the high zeolites (which odors are sometime found in the mouth area (e.g., putrescine, cadaverine, and the like , or originated from food sources, e.g., from sea food). This is important to controlling bad breath. Second, the intermediate zeolites have a larger surface area (700 - 800 m2/g) than the high zeolites (ca. 400 m2/g). This results in more efficient odor adsorptivity, on a wt./wt. basis; or, in the alternative, allows less zeolite to be used to adsorb a given amount of odor. Third, the intermediate zeolites appear to be somewhat more tolerant to moisture, and retain more of their odor-absorbing capacity in the presence of water.

The "intermediate" zeolites used in this invention are characterized by SiO2/AlO2 molar ratios of less than 10. Highly preferred herein the molar ratio of $SiO_2/AlO_2$ will range from 2 to 8.

The synthesis of intermediate zeolites forms no part of the present Invention since various syntheses are known in the extensive zeolite literature.

The following is given simply by way of illustration, and not limitation, of a synthetic procedure.

Synthesis of Special Zeolites

Several post-synthesis modification methods exist for making special zeolites. The methods include (1) pore modification; (2) surface modification and (3) structural change. The first two methods consist of adsorbing species by chemical vapor deposition inside or on the zeolite. Pore modifiers such as $SiH_4$ and $BH_3$ and surface modifiers such as $Si(OCH_4)_4$, $SiCl_4$, $TiCl_4$, and $SeCl_4$ have been used to impart new unique properties to the zeolite. The most frequently used structural change method is to remove alumina from the main framework (i.e., de-aluminate). De-alumination can be performed by one of several routes such as (1) acid leaching; (2) steam (700-900°C); or (3) treatment with $SiCl_4$ at cold temperatures. An example of de-alumination is:

$$Zeolite\ Y + H_4EDTA \rightarrow de\text{-aluminated Zeolite Y}$$

The following references further illustrate the synthesis of intermediate zeolites of the type employed herein: Lok, B. M., Cannan, T. R., and Messing, C. A., "The Role of Organic Molecules in Molecular Sieve Synthesis" Zeolites 3, 282-291 (1983); Barrer, R. M. "Zeolites and their Synthesis" Zeolites 1, 130-140 (1981); ZEOLITES FOR THE NINETIES, Proceedings of the 8th International Zeolite Conference, Eds. P. A. Jacobs and R. A. van Santen (1989) pages 119-372; and MOLECULAR SIEVES, Adv. Chem. Ser. 121, Eds. W. M. Meter and J. B. Uytterhoeven (1973).

A wide variety of intermediate zeolites suitable for use herein are commercially available from commercial suppliers such as Philadelphia Quartz and Conteka. Such materials are sold under various commercial and trade names such as VALFOR CP301-68®, VALFOR 300-63®, VALFOR CP300-35® and VALFOR CP300-56®, from Philadelphia Quartz, and the CBVIOO series (other than Mordenite, as noted above) of zeolites from Conteka.

Another type of odor-absorbing agent, which can be employed in the practice of this invention alone or in combination with the aforesaid intermediate ratio zeolites, comprises the "high ratio" zeolites. Such materials include, for example, the well-known "molecular sieve" zeolites of the ZSM, beta zeolite, etc., type (generally in the 1-10 micron particle size range) and the zeolite materials marketed under the trade name ABSCENTS by the Union Carbide Corporation and UOP, and which are typically available as a white powder in the 3-5 micron particle size range (see: ABSCENTS, A New Approach for Odor Control by A. J. Gioffre, copyright 1988 by the Union Carbide Corporation). Such materials are preferred over the "intermediate" zeolites when control of odors associated with sulfur compounds, e.g., thiols, mercaptans, as well as some control of amine odors, is desired.

The use of zeolites of the ABSCENTS type to control odors is fully described in U.S. Patent 4,795,482, January 3, 1989, to Gioffre and Marcus. In general, these molecular sieve odor-absorbing agents appear to function by entrapping by chemical adsorption odoriferous substances within their molecular lattice structures. Whatever their mode of action, these odor-absorbing agents can be characterized by their physical parameters, as follows. These agents are reported by Gioffre and Marcus to be crystalline siliceous molecular sieves in which at least about 90, and preferably at least about 95, percent of the framework tetrahedral oxide units are $SiO_2$ tetrahedra and which have a sorptive capacity for water at 25°C and 4.6 of less than 10 weight percent.

In the case of aluminosilicate molecular sieves, those "high ratio" zeolite odor-absorbing agents have a framework $SiO_2/AlO_2$ molar ratio of from about 35 to infinity, and preferably from 200 to 500. Such siliceous molecular sieves have a pore diameter of at least 5.5 Angstroms, preferably at least 6.2 Angstroms. Preferably the adsorption capacity for water vapor at 25°C and a water vapor pressure ($p/p_o$) of 4.6 is less than 6 weight percent. As stated by Gioffre and Marcus, the efficacy of these molecular sieves is not dependent on the presence of the water of hydration in the internal cavities of the microporous structure as a result of their hydrothermal formation. In fact, at least a major proportion, usually substantially all, of this original water of hydration is removed in the process of removing any pore-blocking templating agent, which may be present in the adsorbent. Calcination effectively removes any organic moieties. Also, water washing, leaching or washing with a caustic or dilute mineral acid solution is advantageously utilized to remove extraneous synthesis reactants from the pore system. Lowering of the alkali metal content, particularly the nonzeolitic, i.e., occluded alkali metal compounds can also be beneficial. These procedures also serve to remove the original water of hydration.

As further disclosed by Gioffre and Marcus, such siliceous molecular sieves include the microporous crystalline aluminosilicates, i.e., the zeolitic molecular sieves as well as the so-called silica polymorphs. With respect to the latter compositions, their crystal lattices are ideally formed entirely of $SiO_2$ tetrahedral units, but the as-synthesized forms commonly contain at least trace amounts of aluminum derived from aluminum impurities in the synthesis reagents. The aluminosilicate molecular sieves comprise the large class of well-known crystalline zeolites. These high-silica molecular sieves are either commercially available or are prepared by methods well-known in the art, involving direct hydrothermal synthesis or involving certain types of crystal lattice dealuminations. A comprehensive review article by E. M. Flanigen concerning both "high" Si/Al zeolites and silica molecular sieves is published in Proc. 5th Int. Conf. Zeolites, Naples, 1980", L. V. C. Rees, end., Heyden, London, pp. 760- 780. It is to be understood that all such materials are referred to herein simply as "zeolites", for convenience.

With respect to the foregoing ABSCENTS odor-absorbing agents, it is important that their pore system be open so that the internal cavities of the crystals be accessible to the odor molecules. In the case of the aluminosilicates or silica polymorphs produced using large organic templating ions such as tetraalkylammonium ions, it is necessary to remove charge balancing organic ions and any occluded templating material in order to permit adsorption of the odor molecules. In such a removal process and also in the removal of inorganic debris, the original water of hydration is also removed. Upon exposure to the atmosphere, a portion of the water of hydration is reacquired, but this does not affect the characteristics of the molecular sieves which are preferred for the practice of the present invention, i.e., the molecular sieves can be employed in either a hydrated or dehydrated state, but, in general, the dehydrated state is preferred. In the case of most of the dealumination procedures referred to above, the original water of dehydration is also removed, and can similarly be replaced, if desired, for the practice of the invention.

More specifically, Gioffre and Marcus disclose that the class of their disclosed medium to large pore siliceous molecular sieves, from which the original, as-synthesized water of hydration has been substantially removed, and which have a capacity for adsorbed water of not greater than 10, and preferably not greater than 6, weight percent when measured at 25°C and a water vapor pressure ($p/p_o$) of 4.6, function in an extraordinary manner with respect to odor elimination. Many of the synthetic zeolites prepared using organic templating agents are readily prepared in a highly siliceous form - some even from - reaction mixtures which have no intentionally added aluminum. These zeolites are markedly organophilic and include ZSM-5 (U.S. Patent 3,702,886); ZSM-11 (U.S. Patent 3,709,979), ZSM-35 (U.S. Patent 4,016,245); ZSM-23 (U.S. Patent 4,076,842); and ZSM-38 (U.S. Patent 4,046,859) to name only a few. According to these authors, the silica molecular sieves known as silicalite and F-silicalite are particularly suitable for use as odor-absorbing agents. These materials are disclosed in U.S. Patents 4,061,724 and 4,073,865, respectively. To the extent the aforesaid siliceous sieves are synthesized to have $SiO_2/AlO_2$ ratios greater than 35, they are frequently suitable for use in the present inventions without any additional treatment to increase their degree of hydrophobicity. Molecular sieves which cannot be directly synthesized to have both the desired high Si/Al and/or degree of hydrophobicity ratios can be subjected to dealumination techniques, fluorine treatments and the like, which result in organophilic zeolite products. High-temperature steaming procedures for treating zeolite Y which result in hydrophobic product forms are reported by P. K. Maher et al, "Molecular Sieve Zeolites", Advan. Chem. Ser. 101, American Chemical Society, Washington, D.C., 1971, p. 266. A more recently reported procedure applicable to the manufacture of "high" zeolite species generally, involves dealumination and the substitution of silicon into the dealuminated lattice site. This process is disclosed in U.S. Patent 4,503,023 issued March 5, 1985 to Skeels et al. Halogen or halide compound treatments for zeolites to increase their hydrophobicity are disclosed in U.S. Patents 4,569,833 and 4,297,335. Steam-treated zeolite Y, prepared per U.S. Patent 4,331,694, and denominated "LZ-10", is a particularly useful odor-absorbing agent.

Various other modified zeolite-type materials, such as the manganese-aluminum-phosphorus-silicon-oxide molecular sieves described in U.S. Patent 4,793,833, Lok et al, assigned to UOP, can be used herein. See also U.S. Patents 4,604,110; 4,437,429; and 4,648,977, for other zeolitic odor-controlling compositions.

Uncomplexed cyclodextrin and derivatives thereof

These materials are preferred herein due to the unique shape and physical-chemical property of the cavity enable the cyclodextrin molecules to adsorb (form inclusion complexes with) organic molecules or parts of organic molecules which can fit into the cavity.

A preferred odor-absorbing agent for use herein is an uncomplexed cyclodextrin. When referring to cyclodextrin herein for use as an odor-absorbing agent herein it is understood herein uncomplexed cyclodextrin molecules as described herein after.

As used herein, the term "cyclodextrin" includes any of the known cyclodextrins such as unsubstituted cyclodextrins containing from six to twelve glucose units, especially, alpha-cyclodextrin, beta-cyclodextrin, gamma-cyclodextrin and/or their derivatives and/or mixtures thereof. The alpha-cyclodextrin consists of six glucose units, the beta-cyclodextrin consists of seven glucose units, and the gamma-cyclodextrin consists of eight glucose units arranged in a donut-shaped ring. The specific coupling and conformation of the glucose units give the cyclodextrins a rigid, conical molecular structure with a hollow interior of a specific volume. The "lining" of the internal cavity is formed by hydrogen atoms and glycosidic bridging oxygen atoms, therefore this surface is fairly hydrophobic.

Non-derivatised (normal) beta-cyclodextrin is preferred herein. Highly preferred beta-cyclodextrin powder for use in the compositions herein has an average particle size of less than 12 microns, preferably less than 10 to provide the best odor control benefit. The particle size is typically between about 0.001 and 10 microns, preferably between about 0.05 and 5 microns. It is highly desirable that at least an effective amount of particles have the said particle sizes. It is desirable that at least about 50%, preferably at least about 65%, more preferably at least about 80%, of the beta-cyclodextrin powder that is present have the said particle sizes.

These small particles of the invention are conveniently prepared by grinding techniques. Cyclodextrin crystals with large particle sizes can be pulverized to obtain the desired smaller particles of less than about 12 microns by using, e.g., a fluid energy mill. Examples of fluid energy mills are the Trost Air Impact Pulverizers, sold by Garlock Inc., Plastomer Products, Newtown, Pennsylvania; the Micronizer fluid energy mills sold by Sturtevant, Inc., Boston, Massachusetts; and the Spiral Jet Mill sold by Alpine Division, MicroPul Corporation (Hosokawa Micron International, Inc.), Summit, New Jersey.

As used herein, the particle size refers to the largest dimension of the particle and to the ultimate (or primary) particles. The size of these primary particles can be directly determined with optical or scanning electron microscopes. The slides must be carefully prepared so that each contains a representative sample of the bulk cyclodextrin powder. The particles sizes can also be measured by any of the other well-known methods, e.g., wet sieving (non-aqueous), sedimentation, light scattering, etc. A convenient instrument that can be used to determine the particle size distribution of the dry powder directly (without having to make a liquid suspension or dispersion) is the Malvern Particle and Droplet Sizer, Model 2600C, sold by Malvern Instruments, Inc., Southborough, Massachusetts. Some caution should be observed in that some of the dry particles may remain agglomerated. The presence of agglomerates can be further determined by microscopic analysis.

Some other suitable methods for particle size analysis are described in the article "Selecting a particle size analyzer: Factors to consider," by Michael Pohl, published in Powder and Bulk Engineering, Volume 4 (1990), pp. 26 - 29, incorporated herein by reference. It is recognized that the very small particles of the invention can readily aggregate to form loose agglomerates that are easily broken apart by either some mechanical action or by the action of water. Accordingly, particles should be measured after they are broken apart, e.g., by agitation or sonication. The method, of course, should be selected to accommodate the particle size and maintain the integrity of the complex particles with iterative measurements being made if the original method selected proves to be inappropriate.

Particularly preferred cyclodextrins useful in the present invention are highly water-soluble such as, alpha-cyclodextrin and derivatives thereof, gamma-cyclodextrin and derivatives thereof, derivatised beta-cyclodextrins, and/or mixtures thereof. The derivatives of cyclodextrin consist mainly of molecules wherein some of the OH groups are converted to OR groups. Cyclodextrin derivatives include, e.g., those with short chain alkyl groups such as methylated cyclodextrins, and ethylated cyclodextrins, wherein R is a methyl or an ethyl group; those with hydroxyalkyl substituted groups, such as hydroxypropyl cyclodextrins and/or hydroxyethyl cyclodextrins, wherein R is a $-CH_2-CH(OH)-CH_3$ or a $-CH_2CH_2-OH$ group; branched cyclodextrins such as maltose-bonded cyclodextrins; cationic cyclodextrins such as those containing 2-hydroxy-3(dimethylamino)propyl ether, wherein R is $CH_2-CH(OH)-CH_2-N(CH_3)_2$ which is cationic at low pH; quaternary ammonium, e.g., 2-hydroxy-3-(trimethylammonio)propyl ether chloride groups, wherein R is $CH_2-CH(OH)-CH_2-N^+(CH_3)_3Cl^-$; anionic cyclodextrins such as carboxymethyl cyclodextrins, cyclodextrin sulfates, and cyclodextrin succinylates; amphoteric cyclodextrins such as carboxymethyl/quaternary ammonium cyclodextrins; cyclodextrins wherein at least one glucopyranose unit has a 3-6-anhydro-cyclomalto structure, e.g., the mono-3-6-anhydrocyclodextrins, as disclosed in "Optimal Performances with Minimal Chemical Modification of Cyclodextrins", F. Diedaini-Pilard and B. Perly, The 7th International Cyclodextrin Symposium Abstracts, April 1994, p. 49, herein incorporated by reference; and mixtures thereof. Other cyclodextrin derivatives are disclosed in U.S. Pat. Nos: 3,426,011, Parmerter et al., issued Feb. 4, 1969; 3,453,257; 3,453,258; 3,453,259; and 3,453,260, all in the names of Parmerter et al., and all issued July 1, 1969; 3,459,731, Gramera et al., issued Aug. 5, 1969; 3,553,191, Parmerter et al., issued Jan. 5, 1971; 3,565,887, Parmerter et al., issued Feb. 23, 1971; 4,535,152, Szejtli et al., issued Aug. 13, 1985; 4,616,008, Hirai et al., issued Oct. 7, 1986; 4,678,598, Ogino et al., issued Jul. 7, 1987; 4,638,058, Brandt et al., issued Jan. 20, 1987; and 4,746,734, Tsuchiyama et al., issued May 24, 1988; all of said patents being incorporated herein by reference.

Highly water-soluble cyclodextrins are those having water solubility of at least about 10g in 100ml of water at room temperature, preferably at least about 20g in 100ml of water, more preferably at least about 25g in 100ml of water at room temperature. These are easy to use, but are typically more expensive than the non-derivatized beta-cyclodextrin. Examples of preferred water-soluble cyclodextrin derivatives suitable for use herein are hydroxypropyl alpha-cyclodextrin, methylated alpha-cyclodextrin, methylated beta-cyclodextrin, hydroxyethyl beta-cyclodextrin, and hydroxypropyl beta-cyclodextrin. Hydroxyalkyl cyclodextrin derivatives preferably have a degree of substitution of from about 1 to about 14, more preferably from about 1.5 to about 7, wherein the total number of OR groups per cyclodextrin is defined as the degree of substitution. Methylated cyclodextrin derivatives typically have a degree of substitution of from about 1 to about 18, preferably from about 3 to about 16. A known methylated beta-cyclodextrin is heptakis-2,6-di-O-methyl-β-cyclodextrin, commonly known as DIMEB, in which each glucose unit has about 2 methyl groups with a degree of substitution of about 14. A preferred, more commercially available methylated beta-cyclodextrin is a randomly methylated beta-cyclodextrin having a degree of substitution of about 12.6. The preferred cyclodextrins are available, e.g., from Cerestar USA, Inc. and Wacker Chemicals (USA), Inc.

It can be desirable to use a mixture of cyclodextrins. Such mixtures can complex with a wider range of odor molecules having a wider range of molecular sizes. Preferably at least a portion of such a mixture of cyclodextrins is alpha-cyclodextrin or its derivatives, gamma-cyclodextrin or its derivatives thereof, and/or beta-cyclodextrin or its derivatives.

Silica-based material

Particularly suitable herein as an odor absorbing agent is silica. Silica, i.e. silicon dioxide $SiO_2$ exists in a variety of crystalline forms and amorphous modifications, any of which are suitable for use herein. In particular, silicas having a high surface area or in agglomerated forms are preferred (i.e.: clays or shell, etc.). Without being restrictive to a family of silica based materials, commonly the silica is in a highly purified form such that is contains at least 90%, preferably 95%, more preferably 99% silicon dioxide (i.e.: a silica gel having a 100% silica content). Alternatively, the silica may be provided from other sources such as metal silicates including sodium silicate.

Carbon based odor absorbing material

The carbon material suitable for employment herein is the material well known in the art as an absorber for organic molecules and/or air purification purposes. Carbon suitable for use herein is available from a number of commercial sources under the trade names such as CALGON Type "CPG", Type SGL, Type "CAL" and type "OL". Often such material is referred to as "activated" carbon or "activated" charcoal. Typically it is available in the form of an extremely fine, dusty particles (e.g., 0.1-300 microns) having large surface areas (200 - several thousand $m^2$/g.) It is to be understood that any of the "air purifying" or "activated" carbons of commerce can be used in the practice of this invention.

Particles size of the inorganic odor absorbing agents (i.e.: silica-based materials, zeolite-based materials and/or carbon-based materials), is preferably below 30 microns, most preferably less than 10 microns to provide the best feature in odor control benefit associated with feel comfort. The particle size is most preferably between about 0.05 and 5 microns.

Generally talking, the above mentioned odor absorbing agents (such as silica-, zeolite-, carbon-, uncomplexed cyclodextrin-based materials) present very specific absorbing efficiency and capacity towards each odor type (almost always being a mixture of chemicals by itself, and often a mixture of odor types) that in principle is extremely difficult to predict by theory.

This makes very opportune to formulate as a preferred embodiment herein, a chewable composition comprising particular combinations of odor absorbing agents in order to target a broad range of malodorous compounds more effectively.

In practice, the selection of the odor absorbing agents to be used in the chewable compositions according to the present invention (after pre-selection with help of the general principles mentioned in the respective description of the odor absorbing agents) is the result of empirical measurements of the observed efficiency against the targeted malodor bouquet, namely bad breath.

Accordingly in a preferred embodiment herein the chewable compositions comprise combinations of odor absorbing agents, for example uncomplexed cyclodextrin or derivative thereof together with either zeolite-, silica- or carbon-based material, or zeolite-based material together with either silica-based material or carbon-based material and most preferably zeolite-based material together with silica-based material.

As example, an highly preferred odor absorbing system is odor-absorbing agent X together with odor absorbing agent Y in a weight ratio of X to Y in a range of from 1:5 to 5:1, preferably from 3:1 to 1:3 and most preferably about 1:1, with X or Y being respectively an odor absorbing agent selected from the group consisting of silica-, zeolite-, activated carbon-based material and uncomplexed cyclodextrin and derivatives thereof.

Indeed it has been observed that when using X (e.g., zeolite-based material) together with Y (e.g.,silica-based material), provided X and Y belong to two different chemical classes of odor absorbing agents in the above mentioned weight ratio from one to the other, the combination of these odor absorbing agents results in a synergistic odor control towards bad breath.

In another embodiment of the present invention an highly preferred odor absorbing system (for which further improved odor control properties towards bad breath is observed) is odor absorbing agent X together with absorbing agent Y and absorbing agent Z (provided X, Y, and Z belong respectively to 3 different chemical classes of odor absorbing agents), typically in a weight ratio of X, Y and Z in a range of from 1:1:5, 1:5:1 to 5:1:1, preferably from 1:1:3, 1:3:1 to 3:1:1 and most preferably about 1:1:1, with X, Y or Z being respectively an odor absorbing agent selected from the group consisting of silica-, zeolite-, activated carbon-based material and uncomplexed cyclodextrin and derivatives thereof.

Optional odor controlling agents and derivatives thereof

Other odor-controlling agents might be used herein on top of the odor absorbing agents described herein before. Such agents might have different mechanisms of action, including preventing the formation of odor, or controlling the odor once formed and/or masking the odor. All odor-controlling agents known to those skilled in the art might be used herein provided they are safe for oral application. Such odor controlling agents include, but are not limited to, chelating materials, pH buffered materials, antimicrobial agents for example chitin and derivatives thereof, chitosan and derivatives thereof, and the like.

The chewable compositions according to the present invention might comprise from 0.5% to 50% by weight of the total composition of such an odor-controlling agent or mixtures thereof, beside the odor absorbing agent or mixtures thereof, preferably at a level from 2% to 20%.

Optional Cyclodextrin/Active inclusion complexes

In a preferred embodiment herein the chewable compositions might comprise complexed cyclodextrin on top of the odor absorbing agent (e.g., silica, zeolite, carbon and/or uncomplexed cyclodextrin).

Indeed cyclodextrin have the ability to form complexes with other ingredients, so-called 'active' herein. By 'active' it is meant herein any ingredient delivering a desired property/activity to the chewable compositions, including but not limited to, flavoring agents, sweeteners, nutritional or pharmaceutical actives. The cyclodextrin inclusion complexes serve as a carrier for such an active.

In the preferred embodiment of the present invention the active is a flavoring agent. Indeed, the presence of such cyclodextrin/flavoring inclusion complexes, on top of any odor absorbing agent described herein, provides a chewable composition which satisfies the objectives of exhibiting a sufficient degree of flavor extension and initial flavor impact while protecting and even enhancing the stability of the flavoring agent present.

Advantageously it has been found herein that the use of such cyclodextrin/flavoring agent inclusion complexes do not interfere with the efficacy of the odor absorbing agent, thereby providing chewable compositions delivering outstanding odor control of bad breath and a removal signal, per release of such flavoring agent.

The complexes act as a scent signal (via olfactory/taste signal) for the efficiency of the odor absorbing agents present in the chewable compositions. Indeed upon mastication/chewing of the chewable composition the unprotected cyclodextrin/flavoring complex, exhibits flavor release, which phenomenon provides reduction of bad breath (also via malodor/active displacement, or desorption/adsorption) upon longer usage/mastication periods and hence a longer lasting mouth odor control.

The cyclodextrin/active inclusion complexes used herein are very stable in the dry state. Even the very volatile flavouring agent molecules are bound in the cavity of the cyclodextrin molecules and do not provide perceptible odor. Upon wetting by an aqueous fluid such as saliva, the active, preferably flavoring agent, is released to provide a burst of active, namely fragrance. A greater variety of flavouring agents can be used to accommodate a variety of consumer preferences.

Moreover, the encapsulation of actives (preferably flavoring agents) minimizes the interaction with the odor-absorbing agents leading possibly to the inactivation of their benefits, before and during manufacturing process of the chewable composition and storage as well as during use of the composition.

The actives are released when the compositions are wetted (masticated per user), to provide their benefits (i.e.: a pleasant odor-removal signal in use in the case of the active being a flavoring agent). Especially preferred are cyclodextrin inclusion complexes of actives, with a particle size of less than about 12 microns.

In a preferred embodiment, the complexed active is a flavoring agent as described hereafter: the flavoring agents and compositions of this invention are the conventional ones known in the art. Selection of any flavouring agent, or amount of flavouring agents, is based on functional and aesthetic considerations. Preferred flavouring agents useful in the present invention are both natural and artificial flavors and mints, such as oil of peppermint, menthol, oil of spearmint, vanilla, oil of cinnamon, oil of wintergreen (methyl salicylate) and various fruit favors, including but not limited to, orange oil, grape flavor, lime oil, grapefruit oil, apple, apricot essence and combinations thereof. The flavorings are generally utilized in amounts that will vary depending upon the individual flavor and may for example range up to 4% of the total chewable composition.

The formation of the cyclodextrin/active inclusion complexes, namely those wherein the active is a flavoring agent is described hereafter: The flavoring agent/cyclodextrin inclusion complexes of this invention are formed in any of the ways known in the art. Typically, the complexes are formed either by bringing the flavouring agent and the cyclodextrin together in a suitable solvent, e.g., water, or, preferably, by kneading/slurrying the ingredients together in the presence of a suitable, preferably minimal, amount of solvent, preferably water. The kneading/slurrying method is particularly desirable because it produces smaller complex particles and requires the use of less solvent, eliminating or reducing the need to further reduce particle size and separate excess solvent. Disclosures of complex formation can be found in Atwood, J.L., J.E.D. Davies & D.D. MacNichol, (Ed.): Inclusion Compounds Vol. III, Academic Press (1984), especially Chapter 11, Atwood, J.L. and J.E.D. Davies (Ed.): Proceedings of the Second International Svmposium of Cvclodextrins Tokyo, Japan, (July, 1984), and J. Szejtli, Cyclodextrin Technology, Kluwer Academic Publishers (1988), said publications incorporated herein by reference.

In general, flavouring agent/cyclodextrin complexes have a molar ratio of flavouring agent compound to cyclodextrin of about 1:1. However, the molar ratio can be either higher or lower, depending on the size of the flavouring agent compound and the identity of the cyclodextrin compound. The molar ratio can be determined by forming a saturated solution of the cyclodextrin and adding the flavouring agent to form the complex. In general the complex will precipitate readily. If not, the complex can usually be precipitated by the addition of electrolyte, change of pH, cooling, etc. The complex can then be analyzed to determine the ratio of flavouring agent to cyclodextrin.

As stated hereinbefore, the actual complexes are determined by the size of the cavity in the cyclodextrin and the size of the flavouring agent molecule. Desirable complexes can be formed using mixtures of cyclodextrins since flavouring agents are normally mixtures of materials that vary widely in size. It is usually desirable that at least a majority of the material be alpha-, beta-, and/or gamma-cyclodextrin, more preferably beta-cyclodextrin. The content of the flavouring agent in the beta-cyclodextrin complex is typically from about 5% to about 15%, more normally from about 7% to about 12%.

Continuous complexation operation usually involves the use of supersaturated solutions, kneading/slurrying method, and/or temperature manipulation, e.g., heating and then either cooling, freeze-drying, etc. The complexes are dried to a dry powder to make the desired composition. In general, the fewest possible process steps are preferred to avoid loss of flavouring agent.

The cyclodextrin/active inclusion complexes of this invention having a particle size of less than about 12 microns, preferably less than about 10 microns, more preferably less than about 8 microns, and even more preferably less than about 5 microns, improve the release, especially the speed of release of the actives when the complexes are wetted.

The particle size is typically between about 0.001 and 10 microns, preferably between about 0.05 and 5 microns. It is highly desirable that at least an effective amount of the active be in complexes having the said particle sizes. It is desirable that at least about 75%, preferably at least about 80%, more preferably at least about 90%, and even more preferably at least about 100%, of the complex that is present have the said particle sizes. Methods for determining particle sizes have been given hereinbefore.

These small particles of the invention are conveniently prepared by kneading methods and/or grinding techniques. Cyclodextrin complexes with large particle sizes can be pulverized to obtain the desired smaller particles of less than about 12 microns by using, e.g., a fluid energy mill. Examples of fluid energy mills are the Trost Air Impact Pulverizers, sold by Garlock Inc., Plastomer Products, Newtown, Pennsylvania; the Micronizer fluid energy mills sold by Sturtevant, Inc., Boston, Massachusetts; and the Spiral Jet Mill sold by Alpine Division, MicroPul Corporation (Hosokawa Micron International, Inc.), Summit, New Jersey.

In the preferred embodiment herein it is highly desirable that at least an effective amount of the cyclodextrin/active complex be present in the chewable composition. Depending on the type of active used the amount of complexes desired might be different in the chewable composition. Effective amounts of cyclodextrin/favoring agent inclusion complexes are typically in the range of from 0.01% to 10%, preferably from 0.1% to 5%, and more preferably from 0.5% to 3% by weight of the total chewable composition.

Chewable base

According to the present invention the chewable compositions comprise, as an essential component, a chewable base.

The compositions of the present invention can take various forms including chewing gums, chewable tablets and chewing tobaccos. The composition comprises a chewable base appropriate to the desired product form. The chewable base acts to encourage retention of the composition in the oral cavity for a period of few minutes or more, for example by providing an insoluble mass, which does not completely dissolve or disintegrate upon chewing.

Preferably, the compositions of the present invention are in the form of a chewing gum, in which case the chewable base is a chewing gum base as commonly known in the art. Chewing gums are conveniently portable and are often used several times a day. They are typically chewed for several minutes allowing time for active agents to take effect, particularly when, as in the present invention, the active agent can be released over the entire length of the chew.

Chewing gums generally comprise an essentially tasteless, predominantly water insoluble masticatory portion (the gum base) and one or more additives, which may be water-soluble or water-extractable whose purpose is usually to improve the organoleptic properties of the gum. The chewing gum compositions of the present invention generally comprise from 10% to 90%, preferably from 20% to 70%, more preferably 30% to 60%, and most preferably from 25% to 50% of a chewing gum base by weight of the total composition.

An essential ingredient of the chewing gum base is an elastomer or elastomer mixture. Illustrative elastomers include styrene-butadiene rubber, synthetic gums or elastomers such as polyisobutylene and isobutylene-isoprene copolymers; natural gums or elastomers such as chicle, natural rubber, jelutong, balata, gutta-percha, lechicaspi, sorva and mixtures thereof. The elastomer or elastomer mixture is generally present in an amount of from 5% to 30% and preferably from about 7.5% to 25% by weight of the gum base.

An optional but desirable ingredient of the chewing gum base is a resin. The resin serves to plastizise the gum base. Suitable resins for use herein include polyvinyl acetate (PVA) and terpene resins, including polyterpene and polymers of alphapinene or beta-pinene, and mixtures thereof. The resin can conveniently be used at a level of from 5% to 25%, preferably from 8% to 20% by weight of the gum base.

In addition to the resin component, the gum bases useful in the present invention preferably comprise a plasticiser in an amount up to 10%, preferably from 0.1% to 3% by weight of the gum base. Suitable plasticisers include glyceryl triacetate, acetylated monoglyceride, glyceryl tributyrate, ethyl laurate, ethyl acetoacetate, diethyl tartrate, ethyl or butyl lactates, diethyl malate, ethyl oleate, castor oil, succinylated monoglycerides or mixtures thereof. Glyceryl triacetate and acetylated monoglyceride are preferred.

Various fats can also be included in the gum base. Preferred fats include the hydrogenated vegetable oils such as hydrogenated palm oil, hydrogenated soybean oil, hydrogenated cotton seed oil and various other hydrogenated vegetable oils and mixtures thereof. The fats can suitably be used at a level up to about 20%, preferably from about 1 % to about 10% by weight of the gum base.

A further desirable ingredient of the chewing gum base is an elastomer solvent. The elastomer solvent aids in softening the elastomer component. Such elastomer solvents include methyl, glycerol or pentaerythritol esters of rosins or modified rosins, such as hydrogenated, dimerized or polymerised rosins or mixtures thereof.

Examples of elastomer solvents suitable for use herein include the pentaerythritol ester of partially hydrogenated wood rosin, pentaerythritol ester of wood rosin, glycerol ester of partially dimerized rosin, glycerol ester of polymerised rosin, glycerol ester of tall oil, wood or gum rosin, glycerol ester of partially hydrogenated rosin, methyl ester of partially hydrogenated rosin, and mixtures thereof. The elastomer solvent can be employed in an amount ranging from 2% to 50%, preferably from 10% to 3 5% by weight of the gum base.

The gum bases can also include one or more waxes. Suitable waxes include paraffin wax; n-ticrocrystalline wax; Fischer-Tropsch paraffin; -natural waxes such as candellilla, carnauba and beeswax; polyolefin waxes such as polyethylene wax; and mixtures thereof. The waxes can be present in levels up to 25%, preferably from 5% to 20% by weight of the gum base.

The gum base also preferably includes an emulsifier. Suitable emulsifiers include glycerol monostearate, lecithin, fatty acid monoglycerides, diglycerides, propylene glycol monostearate and mixtures thereof. The emulsifier is employed in amounts up to 10% and preferably from 2% to 6% by weight of the gum base.

A variety of softeners can also be employed in the gum bases useful in the present invention. Suitable softeners include fatty materials such as lanolin, stearic acid, sodium stearate and potassium stearate; polyhydric alcohols such as glycerine, sorbitol and the like; and mixtures thereof. The softeners can suitably be used at a total level of up to 3 0%, preferably from 0. 1% to 10% by weight of the gum base. Preferably, the fatty softener is stearic acid. Such materials, when incorporated into the gum base, assist in modifying the texture and consistency properties. In particular, they help to soften the chew and to maintain chew softness over an extended period of time.

Bulking agents, such as fillers, can also be employed in the gum base. Suitable fillers and bulking agents are generally non-abrasive, preferably with an average particle size less than 5 microns, more preferably less than 3 microns and especially less than 1 microns.

Illustrative bulking agents include calcium carbonate or ground limestone, talc, aluminium hydroxide, alumina, aluminium silicates, dicalcium phosphate and mixtures thereof. Where present, the filler can be used in levels up to 50%, preferably up to 30%, most preferably from up to 20% by weight of the gum base.

In preferred embodiments, the gum base further comprises a high intensity sweetener. Suitable high intensity sweeteners include: dipeptide based sweeteners such as Laspartyl-L-phenylalanine methyl ester (Aspartame) and equivalents described in U.S. Pat. No. 3,492,131, L-(x-aspartyl-N-(2,2,4,4-tetramethyl-3-thietanyl)-D-alaninan de hydrate (Alitame) and the like; the soluble saccharin salts, i.e., sodium or calcium saccharin salts; cyclamate salts, acesulfame-K and the like; the free acid form of saccharin; chlorinated derivatives of sucrose such as chlorodeoxysucrose and the like; and protein based sweeteners, such as Thaurnatin (talin). The high intensity sweeteners described can be added in amounts of from 0.01% to 2.0% and most preferably from 0.05% to 0.5% by weight of the gum base.

Using a high intensity sweetener within the gum base prolongs the flavor of the finished gum composition during chewing.

In addition, the gum base can also include colorants and pigments, such as titanium dioxide. In general, the gum base can contain up to about 2% of pigment and / or colorant. Anti-oxidants can also be included in the gum base, at a level of up to 0.5%. Suitable anti-oxidants are butylated hydroxyanisole, butylated hydroxytoluene, propyl gallate, ascorbic acid and tocopherols.

Finished gum bases are commercially available.

The chewing gum compositions of the present invention generally further comprise various organoleptic compounds, typically at levels of from 15% to 80%, preferably from 20% to 65%, and more preferably from 25% to 50% by weight of the total composition. As used herein, the term 'organoleptic compounds' means those ingredients, which are added to the prepared gum base to modify the aesthetic appreciation of the final chewing gum composition by the consumer, but does not include the various forms of active agent described above. Such ingredients have the principal purpose of providing flavor and sweetness to the gum, and of modifying and enhancing the gum's chewing characteristics or other use properties, such as tackiness or initial hardness. A variety of organoleptic additives can be used, including bulk and high intensity sweeteners, flavourants, softeners, and fillers.

Suitable bulk sweeteners are monosaccharides, disaccharides, and polysaccharides such as xylose, ribose, glucose, mannose, galactose, fructose, dextrose, sucrose, sugar maltose, flucto oligo saccharide syrups, partially hydrolysed starch, or corn syrup solids and sugar alcohols such as sorbitol, xylitol, mannitol, maltitol and mixtures thereof. Preferably the sugar alcohols are used since they are noncariogenic.

Suitable high intensity sweeteners are those described above as optional gum base ingredients.

In general, the amount of sweetener will vary with the sweetener used and desired amount of sweetener selected for a particular chewing gum. This amount will normally vary from 0.01% when using a high intensity sweetener to 80% by weight of the chewing gum composition when using an easily extractable bulk sweetener. The bulk sweeteners described above, are preferably used in amounts of 30% to 70% by weight and most preferably 35% to 60% by weight. By contrast, the high intensity sweeteners described are used in amounts of 0.01% to 2.0% and most preferably 0.05% to 0.5% by weight of the final gum composition. These amounts are ordinarily necessary to achieve a desired level of sweetness independent from the flavor level achieved from the flavoring agents.

Flavoring agents well known in the chewing gum art can optionally be added to the chewing gum compositions of the invention. These flavoring agents can be chosen from synthetic flavoring liquid and/or oils derived from plants leaves, flowers, fruits and so forth, and combinations thereof. Representative flavoring liquids include: spearmint oil, cinnamon oil, oil of wintergreen (methylsalicylate) and peppermint oils.

Also useful are artificial, natural or synthetic fruit flavors such as citrus oil including lemon, orange, banana, grape, lime, apricot and grapefruit and fruit essences including apple, strawberry, cherry, orange, pineapple and so forth; bean and nut derived flavors such as coffee, cocoa, cola, peanut, almond and so forth.

The amount of flavoring agent employed is normally a matter of preference subject to such factors as flavor type, base type and strength desired. In general, amounts up to 4% by weight and preferably 0.05% to 3% by weight of the final chewing gum composition are usable with amounts of 0.8% to 2.5% being preferred.

Softeners can optionally be included in the chewing gum composition to improve the chew characteristics and mouth feel of the gum. The softeners will generally constitute from about 0.5% to about 15% by weight of the chewing gum composition and can include glycerine, lecithin, and mixtures thereof.

The chewing gum composition of this invention can additionally comprise other conventional additives inclusive of coloring agents such as titanium dioxide; emulsifiers such as lecithin and glyceryl monostearate; and fillers as described above, for example, dicalcium phosphate, aluminium hydroxide, and combinations thereof.

The total amount of fillers present is generally up to 10% by weight of the final composition.

Manufacturing methods

All forms of odor absorbing agents for use in the present invention can be incorporated into the compositions of the invention using methods well-known in the art.

A special attention is required for the preparation protocol as this may influence greatly the final result of the chewing gum features both from a chewing gum texture and quality point as well as from the odor control efficiency and process (i.e.: for controlled release of odor absorbing agents in the mouth during mastication/chewing). For example, if a little or even no release of the odor absorbing agents is desired in the mouth, the odor absorbing agents will be mixed at an early stage of the preparation of the chewing gum (typically during the preparation of the gum base). In contrast if some to most of the absorbing agents release is desired in the mouth, the absorbing agents will be mixed at a latest stage of the preparation of the chewing gum, typically to the gum base.

Importantly as well, it may be very much desirable in some instance to externally coat the odor absorbing agents with some water soluble materials (including but not limited to sugar, polyols, polymeric materials) in order to form a protective layer which upon mastication would dissolve/displace and hence set free the absorbing agents to fully operates. This is desirable in preferred embodiment herein especially to prevent potential interaction that might occur between the odor absorbing agents and some of the ingredients of the chewing gum, during the preparation or the storage of the chewing gum composition, thereby preventing potential alteration of the chewing gum texture or properties and/or the malodor control efficiency or kinetic.

It is preferred to add the odor absorbing agents at a process temperature of less than 70°C, preferably less than 60°C, more preferably less than 55°C to avoid causing interaction or degradation of the ingredients or their properties.

The general techniques for manufacturing confectionery products of the type described herein can be found in "Skuse's Complete Confectioner", 13th Edition, 1957, published by W.J. Bush & Company Ltd, referred to above. A more up to date source giving fuller detail of suitable equipment is the "Silesia Confiserie Manual No. 3", published by Silesia-Essenzenfabrik Gerhard Hanke K.G., Abt. FachbOcherei.

The following examples are given to illustrate the compositions according to the invention. However, the invention is not limited thereto.

Table I describes some examples of chewing gum compositions according to the invention (see below).

The chewing gum compositions are prepared by:

- Step 1: Softening the commercially available gum base per by gentle warming and transfer the gum base into a kettle

- Step 2: adding mannitol powder and mixing for approximately 5 minutes until an homogeneous mixture is obtained.

- Step 3: To this mixture, 2/3 of the sorbitol is slowly added during the mixing process, which lasts for about 10 minutes.

- Step 4: To this mixture maltitol syrup is added and mixing is continued for about 3 minutes.

- Step 5: The remaining third of the sorbitol is added, and mixing continued for about 5 minutes.

- Step 6: Then the glycerine, spray-dried menthol, hydrophobically coated menthol, aspartame, fat and peppermint oil, are added by turn, mixing after each additional ingredient for about 2 minutes.

- Step 7: The gum is discharged from the kettle, formed into chunks and conditioned to room temperature (24"C).

| *Additive composition (W/W): | | |
|---|---|---|
| | Peppermint oil | 3.2 |
| | Spray-dried menthol (1) | 4.5 |
| | Hydrophobically coated menthol (2) | 7.5 |
| | Sorbitol | 55 |
| | Fat(3) | 1 |
| | Mannitol | 10.5 |
| | Maltitol syrup (4) | 10.5 |
| | Glycerine | 7.5 |
| | Aspartame | 0.3 |
| | Total (w/w percent) | 100 |

(1) A water-releasable, entrapped menthol consisting essentially of 15:85 menthol:gum acacia

(2) A hydrophobically coated menthol consisting essentially of 8:48:44 menthol:gurn acacia:hardened palm oil, prepared according to GB-A-1,327,761. The hardened palm oil has a melting point of 55-57°C.

(3) Hardened palm oil having a melting point of 55-57°C

(4) Lycasin, 85% solids, a product commercially available from Roquette

## Table I

| Chewing gum composition (w/w percent per total chewing gum composition) protocol → type of odor absorbing agent ↓ | Type and % of gum base | % additives* | % cyclodextrin/menthol complex (90/10 w/w %) | % odor absorbing agent | Addition of odor absorbing agent in the process described herein before |
|---|---|---|---|---|---|
| Zeolite Na-5-ZSM from DEGUSSA Particle size (0.5-5 microns) | Prestige PL228/01 filler free from Cafosa, Spain 27% | 66 % | 0% | 7% | Before step 2, and mix 20 min. |
| Beta Cyclodextrin from FLUKA | P298/96 from Cafosa 30% | 60 % | 1% | 9% | After step 6, and mix 5 min. |
| Silica shell amorphous Zelec HX1233 from Dupont (50%) + Zeolite Na-5-ZSM from DEGUSSA (50%) | Prestige PL228/01 filler free from Cafosa 27% | 65 % | 1% | 7% | Before step 2, and mix 20 min. |
| Silica shell amorphous Zelec HX1233 from Dupont (50%) + Beta Cyclodextrin from FLUKA (50%) | P298/96 filler free from Cafosa 21% | 66 % | 0% | 13% | After step 6, and mix 5 min. |
| Activated Charcoal (05117) from Fluka (33%) + Beta Cyclodextrin from FLUKA (33%) + Silica shell amorphous Zelec HX1233 from Dupont (33%) | Prestige PL228/01 filler free from Cafosa 24% | 66 % | 0% | 10% | Before step 2, and mix 20 min. |

Note: odor absorbing agents were sieved below 10 microns (except indicated)

## Claims

1. A chewable composition comprising a chewable base and an odor absorbing agent.

2. A chewable composition according to claim 1, wherein the odor absorbing agent is selected from the group consisting of zeolite-based material, silica-based material, uncomplexed cyclodextrin and derivative, carbon-based odor-absorbing agent and mixture thereof, and most preferably is a mixture of such odor absorbing agents or zeolite-base material alone or cyclodextrin or derivative alone.

3. A chewable composition according to any of the preceding claims, wherein the absorbing agent is a mixture of cyclodextrin or derivative thereof together with either silica-, zeolite-based material, and/or carbon-based odor absorbing agent, or a mixture of zeolite-based material together with silica-and/or carbon-based material.

4. A chewable composition according to any of the preceding claims wherein the odor absorbing agent is a mixture of (a) a first odor absorbing agent selected from the group consisting of zeolite-based material, silica-based material, uncomplexed cyclodextrin and derivative thereof, and carbon-based odor absorbing material, together with (b) a second odor absorbing agent selected from the group consisting of zeolite-based material, silica-based material, uncomplexed cyclodextrin and derivative thereof, and carbon-based odor absorbing material, provided (a) and (b) belong to two different chemical classes of odor absorbing agents, preferably at a weight ratio of (a) to (b) of from 1:5 to 5:1, more preferably from 1:3 to 3:1 and most preferably about 1:1.

5. A chewable composition according to claim 4, wherein (a) is a zeolite-based material and (b) is a silica-based material.

6. A chewable composition according to any of the preceding claims which is preferably a chewing gum composition comprising such an odor absorbing agent or mixture thereof at a level from 1 to 70% by weight of the total composition, preferably from 3% to 30% and more preferably from 5% to 15%.

7. A chewable composition according to any of the preceding claims which is preferably a chewing gum composition comprising from 10% to 90% of chewing gum base by weight of the total composition, preferably from 20% to 70%, and most preferably from 25% to 50%.

8. A chewable composition according to any of the preceding claims which further comprises a cyclodextrin/active inclusion complex, preferably a cyclodextrin/flavoring agent inclusion complex.

9. A chewable composition according to claim 8, which is preferably a chewing gum composition, comprising from 0.01% to 10%, and preferably from 0.5% to 3% of cyclodextrin/flavoring agent inclusion complex by weight of the total composition.

10. The use, in a chewable composition, typically a chewing gum, of an odour absorbing agent or combination thereof, for effective reduction of bad breath.

**European Patent Office**

## EUROPEAN SEARCH REPORT

Application Number

EP 01 11 9703

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 59428 A (FUISZ INTERNATIONAL LTD) 25 November 1999 (1999-11-25) * page 1, line 10-15 * * page 8, line 27-31 * | 1-10 | A23G3/30 |
| X | US 4 167 559 A (MICHEL GEORGE H) 11 September 1979 (1979-09-11) * column 1, line 16; claim 1 * | 1 | |
| X | US 6 174 514 B1 (CHAU TOMMY L ET AL) 16 January 2001 (2001-01-16) * column 2, line 45-67 * * column 4, line 59 - column 5, line 58 * | 1-10 | |
| | | | **TECHNICAL FIELDS SEARCHED (Int.Cl.7)** A23G |

The present search report has been drawn up for all claims

| Place of search | Date of completion of the search | Examiner |
|---|---|---|
| MUNICH | 14 January 2002 | Weber, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**

EP 01 11 9703

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2002

| Patent document cited in search report | | Publication date | Patent family member(s) | | Publication date |
|---|---|---|---|---|---|
| WO 9959428 | A | 25-11-1999 | US | 6174514 B1 | 16-01-2001 |
| | | | AU | 3660499 A | 06-12-1999 |
| | | | EP | 1079702 A1 | 07-03-2001 |
| | | | WO | 9959428 A1 | 25-11-1999 |
| US 4167559 | A | 11-09-1979 | GB | 1561374 A | 20-02-1980 |
| | | | JP | 1355110 C | 24-12-1986 |
| | | | JP | 53133645 A | 21-11-1978 |
| | | | JP | 61019230 B | 16-05-1986 |
| US 6174514 | B1 | 16-01-2001 | AU | 3660499 A | 06-12-1999 |
| | | | EP | 1079702 A1 | 07-03-2001 |
| | | | WO | 9959428 A1 | 25-11-1999 |

EPO FORM P0459

For more details about this annex : see Official Journal of the European Patent Office, No. 12/82